# EUROPEAN PATENT APPLICATION

(11) **EP 1 407 768 A2**
(43) Date of publication of application: **14.04.2004**
(21) Application number: 03078618.0
(22) Date of filing: 30.06.2000
(51) Int. Cl.: A61K 31/27

(54) **The potassium channel opener retigabine for the treatment of diseases**

(30) Priority: 01.07.1999 GB 9915414
(62) Divisional of application: 00940669.5
(71) Applicant: GLAXO GROUP LIMITED, Greenford, Middlesex UB6 ONN (GB)
(72) Inventor: Burbidge, Stephen Anthony, Stevenage Hertfordshire SG1 2NY (GB); Clare, Jeffrey John, Stevenage Hertfordshire SG1 2NY (GB); Cox, Brian, Stevenage Hertfordshire SG1 2NY (GB); Dupere, Joseph, Cranfield Bedfordshire MK43 0TD (GB); Hagan, Russell Michael, Research Triangle Park NC 27709 (US); Xie, Xinmin, Burlingame CA 94010 (US)
(74) Representative: Hockley, Siân Catherine

(57) **Abstract**

The present invention relates to novel uses for retigabine and KCNQ2/3 potassium channel openers.

## Description

The present invention relates to new uses for the compound retigabine and new uses for KCNQ2/3 potassium channel openers.

Retigabine, 2-amino-4-(4-fluorobenzylamino)-1-ethoxycarbonylaminobenzene. is a known anti-convulsant compound described for example in US5,384,330. The mechanism of action of retigabine is not known, but there are reports of activity against a potassium channel expressed in PC12 and NG108-15 cells. Retigabine has also been reported to increase *de novo* GABA synthesis in neurones and act at piperidic acid A receptors.

It has now been found that retigabine is a KCNQ2/3 potassium channel opener and retigabine's anti-convulsant activity is due to its activity at these channels.

KCNQ2 and KCNQ3 together form a heterotetrameric nervous system-specific potassium channel described for example in WO99/07832. WO99/07832 suggests utilities for modifiers of KCNQ2/3 channels including neurotransmission, ataxia, myokymia, seizures (e.g. epileptic seizures), Alzheimer's disease, Parkinson's disease, age-associated memory loss, learning deficiencies, motor neuron diseases and stroke. However, there is no teaching as to whether KCNQ2/3 openers or dosers would be of use in the above diseases and no *in vitro* or *in vivo* support for these suggestions. WO99/07832 does not disclose any compounds active at KCNQ2/3 receptors. Linopirdine is a known compound which is a selective KCNQ2/3 blocker with cognition enhancing activity.

It has now been found that KCNQ2/3 potassium channel openers are useful as, amongst others, analgesics, fever reducers, musde relaxants, anxiolytics and are of use in migraine, bipolar disorder and unipolar depression.

The invention accordingly provides, in a first aspect, the novel use of retigabine as a KCNQ2/3 potassium channel opener.

There is also provided as a further aspect of the invention the use of retigabine in the preparation of a medicament for use in the treatment of conditions ameliorated by KCNQ2/3 potassium channel opening.

In an alternative or further aspect there is provided a method for the treatment of a mammal, including man, suffering from or susceptible to conditions ameliorated by KCNQ2/3 potassium channel opening, comprising administration of an effective amount of retigabine.

In a further aspect there is provided the use of retigabine in the preparation of a medicament for: use in neurotransmission disorders; CNS disorders; functional bowel disorders; neurodegenerative diseases; neuroprotection; tinnitus; preventing or reducing dependence on, or preventing or reducing tolerance or reverse tolerance to, a dependence - inducing agent; cancerous diseases; inflammatory processes; ophthalmic diseases; cognitive disorders; and migraine; and use especially as a centrally acting analgesic.

In a yet further aspect the invention provides a method for: the treatment of neurotransmission disorders, CNS disorders, functional bowel disorders, neurodegenerative diseases, or tinnitus; preventing or reducing dependence on, or preventing or reducing tolerance or reverse tolerance to, a dependence - inducing agent; the treatment of cancerous diseases, inflammatory processes, ophthalmic diseases, cognitive disorders, or migraine; and producing a neuroprotecting, or more especially a centrally acting analgesic effect; in a mammal, including man, comprising administration of an effective amount of retigabine.

In a further aspect the invention provides the use of a KCNQ2/3 potassium channel opener in the preparation of a medicament for: use as a muscle relaxant, fever reducer, or anxiolytic; use in migraine, bipolar disorder, unipolar depression, functional bowel disorders, or tinnitus; use in preventing or reducing dependence on, or preventing or reducing tolerance or reverse tolerance to, a dependence - inducing agent; use in cancerous diseases, inflammatory processes, or ophthalmic diseases; and use especially as an analgesic.

In a further aspect the invention provides a method: of producing a muscle relaxant, fever reducing, or anxiolytic effect; for treating migraine, bipolar disorder, unipolar depression, functional bowel disorders, or tinnitus; for preventing or reducing dependence on, or preventing or reducing tolerance or reverse tolerance to, a dependence - inducing agent; for treating cancerous diseases, inflammatory processes, or ophthalmic diseases; and especially of producing an analgesic effect; in a mammal, including man, comprising administration of an effective amount of a KCNQ2/3 potassium channel opener.

In a further aspect the invention provides the use of a KCNQ2/3 potassium channel opener in the preparation of a medicament for use as an anti-epileptic.

In a further aspect the invention provides a method for the treatment of a mammal, including man, suffering from or susceptible to epilepsy, comprising administration of an effective amount of a KCNQ2/3 potassium channel opener.

According to the instant invention KCNQ2/3 potassium channel openers including retigabine are useful as anticonvulsants. They are therefore useful in treating epilepsy. They may be used to improve the condition of a host, typically a human being, suffering from epilepsy. They may be employed to alleviate the symptoms of epilepsy in a host. "Epilepsy" is intended to include the following seizures:- simple partial seizures, complex partial seizures, secondary generalised seizures, generalised seizures including absence seizures, myoclonic seizures, clonic seizures, tonic seizures, tonic clonic seizures and atonic seizures.

KCNQ2/3 potassium channel openers including retigabine are additionally useful in the treatment of CNS disorders such as bipolar disorder, alternatively known as manic depression. Type I or II bipolar disorder may be treated. The compounds may thus be used to improve the condition of a human patient suffering from bipolar disorder. They may be used to alleviate the symptoms of bipolar disorder in a host. The compounds may also be used in the treatment of unipolar depression, ataxia, myokimia and anxiety.

KCNQ2/3 potassium channel openers including retigabine are also useful as analgesics. They are therefore useful in treating or preventing pain. They may be used to improve the condition of a host, typically a human being, suffering from pain. They may be employed to alleviate pain in a host. Thus, the compounds may be used as a pre-emptive analgesic to treat acute pain such as musculoskeletal pain, post operative pain and surgical pain, chronic pain such as chronic inflammatory pain (e.g. rheumatoid arthritis and osteoarthritis), neuropathic pain (e.g. post herpetic neuralgia, trigeminal neuralgia and sympathetically maintained pain) and pain associated with cancer and fibromyalgia. The compounds may also be used in the treatment or prevention of pain associated with migraine. The compounds may also be used in the treatment of the pain (both chronic and acute), fever and inflammation of conditions such as rheumatic fever, symptoms associated with influenza or other viral infections, such as the common cold; lower back and neck pain; headache; toothache; sprains and strains; myositis; neuralgia; synovitis; arthritis, including rheumatoid arthritis; degenerative joint diseases, including osteoarthritis; gout and ankylosing spondylitis; tendinitis; bursitis; skin related conditions, such as psoriasis, eczema, bums and dermatitis; injuries, such as sports injuries and those arising from surgical and dental procedures.

KCNQ2/3 potassium channel openers including retigabine are further useful in the treatment of functional bowel disorders which include non-ulcer dyspepsia, non-cardiac chest pain and in particular irritable bowel syndrome. Irritable bowel syndrome is a gastrointestinal disorder characterised by the presence of abdominal pain and altered bowel habits without any evidence of organic disease. The compounds may thus be used to alleviate pain associated with irritable bowel syndrome. The condition of a human patient suffering from irritable bowel syndrome may thus be improved.

KCNQ2/3 potassium channel openers including retigabine are also useful in the treatment of neurodegenerative diseases, such as Alzheimer's disease, ALS, motor neuron disease, Parkinson's disease, macular degeneration and glaucoma. The compounds may also be useful in neuroprotection and in the treatment of neurodegeneration following stroke, cardiac arrest, pulmonary bypass, traumatic brain injury, spinal cord injury or the like.

KCNQ2/3 potassium channel openers including retigabine are further useful in the treatment of tinnitus.

KCNQ2/3 potassium channel openers including retigabine are additionally useful in the treatment of migraine.

Still further, KCNQ2/3 potassium channel openers including retigabine are also useful in preventing or reducing dependence on, or preventing or reducing tolerance or reverse tolerance to, a dependence - inducing agent. Examples of dependence inducing agents include opioids (e.g. morphine), CNS depressants (e.g. ethanol), psychostimulants (e.g. cocaine) and nicotine.

KCNQ2/3 potassium channel openers including retigabine may inhibit cellular and neoplastic transformation and metastatic tumour growth and hence be useful in the treatment of certain cancerous diseases, such as colonic cancer.

KCNQ2/3 potassium channel openers including retigabine may inhibit inflammatory processes and therefore may be of use in the treatment of asthma, allergic rhinitis and respiratory distress syndrome; gastrointestinal conditions such as inflammatory bowel disease, Chron's disease, gastritis, irritable bowel syndrome and ulcerative colitis; and the inflammation in such diseases as vascular disease, migraine, periarteritis nodosa, thyroiditis, aplastic anemia, Hodgkin's disease, sclerodoma, type I diabetes, myasthenia gravis, multiple sclerosis, sorcoidosis, nephrotic syndrome, Bechet's syndrome, polymyositis, gingivitis, conjunctivitis and myocardial ischemia.

KCNQ2/3 potassium channel openers including retigabine may also be useful in the treatment of ophthalmic diseases such as retinitis, retinopathies, uveitis and of acute injury to the eye tissue.

KCNQ2/3 potassium channel openers including retigabine may also be useful for the treatment of cognitive disorders such as dementia, particularly degenerative dementia (including senile dementia, Atzheimer's disease, Pick's disease, Huntington's chorea, Parkinson's disease and Creutzfeldt-Jakob disease), and vascular dementia (including multi-infarct dementia), as well as dementia associated with intracranial space occupying lesions, trauma, infections and related conditions (including HIV infection), metabolism, toxins, anoxia and vitamin deficiency; and mild cognitive impairment associated with ageing, particularly Age Associated Memory Loss; and learning deficiencies.

It will be appreciated that reference to treatment is intended to include prophylaxis as well as the alleviation of established symptoms.

The KCNQ2/3 potassium channel opening activity of retigabine has been illustrated by the following method.

### Expression of KCNQ2 & KCNQ3 in Xenopus oocytes: Modulation by Retigabine.

Human KCNQ2 and KCNQ3 potassium channels were cloned from a human brain CDNA library using standard methodology. All clones were sequenced and were identical to those reported in the literature (Charlier, C., Singh, N.A., Ryan, S.G., Lewis, T.B., Reus, B. E., Leach, R.J. Leppert, M. (1998). A pore mutation in a novel KQT-like potassium channel gene in an idiopathic epilepsy family. *Nature Genetics* **18**: 53-55 and Singh, N.A., Charlier, C., Stauffer, D., DuPont, B.R., Leach, R.J., Melis, R., Ronen, G.M., Bjerre, I., Quattlebaum, T., Murphy, J.V., McHarg, M.L., Gagnon, D., Rosales, T.O., Peiffer, A., Anderson, E & Leppert, M. (1998) *Nature Genetics*, **18:** 25-29).

Adult female *Xenopus laevis* (Blades Biologicals) were anaesthetised using 0.2% tricaine (3-aminobenzoic acid ethyl ester), killed and the ovaries rapidly removed. Oocytes were de-folliculated by collagenase digestion (Sigma type I, 1.5 mg ml⁻¹) in divalent cation-free OR2 solution (82.5mM NaCl, 2.5mM KCI, 1.2mM NaH₂PO₄, 5mM HEPES; pH 7.5 at 25°C). Single stage V and VI oocytes were transferred to ND96 solution (96mM NaCl, 2mM KCI, 1mM MgCl₂, 1.8mM CaCl₂, 5mM HEPES; pH 7.5 at 25°C) which contained 50µg ml⁻ ¹ gentamycin and stored at 18°C.

KCNQ2 (in pCIN3 vector) and KCNQ3 (in pCIH6) were linearised and transcribed to RNA using T7 or T3 polymerase (Promega Wizard kit). m'G(5')pp(5')GTP capped cRNA was injected into oocytes (20-50nl of 1µgµl⁻¹ RNA per oocyte) and whole-cell currents were recorded using two-microelectrode voltage-clamp (Geneclamp amplifier, Axon instruments Inc.) 3 to 5 days post-RNA injection. Microelectrodes had a resistance of 0.5 to 2MΩ when filled with 3M KCI. In all experiments oocytes were voltage-clamped at a holding potential of -90mV in ND96 solution (superfused at 2ml per min.) and test compounds were applied by addition to this extracellular solution. Current-voltage curves were constructed by applying 800ms voltage-clamp pulses from the holding potential of -90mV to test potentials between -85mV and +30mV..

Whole-cell currents were recorded from oocytes expressing KCNQ2 alone, KCNQ3 alone and an equimolar combination of KCNQ2 plus KCNQ3. Mean current amplitude (at +20mV) was 152 +/- 21 nA for KCNQ2 alone, 157 +/-19nA for KCNQ3 alone and 1467 +/- 420nA for KCNQ2 & KCNQ3. Further characterisation of the KCNQ2/KCNQ3 heteromeric channel (current-voltage curve, conductance plot, linopirdine block) gave similar results to those reported in the literature (Yang, W.-P., Levesque, P.C., Little, W.A., Conder, M.L., Ramakrishnan, P., Neubauer, M.G. & Blanar, M.A, (1998) Functional expression of two KvLQT1-related potassium channels responsible for an inherited idiopathic epilepsy. *J. Biol. Chem.,* **31**: 19419-19423 and Wang, H.-S., Pan, Z., Shi, W., Brown, B.S.,. Wymore, R.S., Cohen, I.S., Dixon, J.E. & McKinnon, D (1998) KCNQ2 and KCNQ3 potassium channel subunits: molecular correlates of the M-channel. *Science* **282**: 1890-1893).

The effects of retigabine were studied in oocytes expressing equimolar KCNQ2 plus KCNQ3. Application of 10µM retigabine led to a pronounced 20mV hyperpolarising shift in the threshold for KCNQ current activation (n=5), such that an augmentation of KCNQ current was seen over the normal physiological range of membrane potentials. For example, at a test potential of -60mV, outward KCNQ current amplitude increased 8-fold in the presence of retigabine (6.2-fold at -70mV; 7.1-fold at -50mV; n=5). These changes in KCNQ current amplitude were associated with an increase in the rate of current activation: Retigabine increased the rate of current activation 15-fold at a test potential of -40mV, and 3-fold at a test potential of -30mV (data fitted to a single exponential in each case).

Application of retigabine led to a marked alteration in the properties of the KCNQ2/3 tail currents. In these experiments the voltage clamp protocols comprised a 1s prepulse from -100mV to +40mV to fully activate the KCNQ2/3 channel, followed by a 6s pulse to test potentials between -30mV and -110mV. At the most positive test potentials (-30mV, -40mV) a steady state KCNQ2/3 tail current was recorded during the test pulse, suggesting that deactivation does not occur at these test potentials. With subsequent pulses to less positive membrane potentials (-50mV to -110mV) channel deactivation was observed but the rate was significantly slowed in comparison to control current data. These results suggest that in the presence of retigabine, the voltage dependence of channel deactivation shifts to more hyperpolarised membrane potentials.

Further experiments were carried out in oocytes held under current clamp. In these experiments the retigabine-induced shift in KCNQ activation curve led to a hyperpolarisation of the cell. The half maximal inhibitory concentration for this hyperpolarisation was 5.2µM (95% confidence limits 3.9-7.0µM) and the Hill slope was 1.1 ± 0.1.

In conclusion, we have shown that retigabine opens KCNQ2/3 potassium channels, apparently through an increase in the kinetics of channel activation. Since KCNQ2 and KCNQ3 are widely and prominently expressed throughout the central nervous system, the properties of retigabine on the KCNQ2/3 channel are likely to be a major contributor of its anti-convulsant action in vivo.

### Expression of KCNQ2 in Xenopus oocytes: Modulation by Retigabine.

Using a specialist ooctye expression vector, pSP64t (Kreig PA and Melton DA (1984) Functional messenger RNAs are produced by SP6 in vitro translation of cloned cDNAs. Nucleic Acids Research 12:7057-7070) we were able to successfully record currents from oocytes expressing KCNQ2 alone. Application of 10µM retigabine led to a pronounced 20mV hyperpolarising shift in the threshold for KCNQ2 current activation, increased KCNQ current amplitude over a range of test potentials (control = 145+/-34nA; retigabine = 501+/-116nA at -50mV, n=7) and increased the rate of current activation (mean delay at -40mV: control 132+/-16ms; retigabine 75+/-7ms). This data suggests that at least a part of retigabine's actions on the KCNQ2/3 heteromer occurs through an interaction with the KCNQ2 channel.

In addition to the anti-convulsant properties of retigabine where the drug protected against pentylene tetrazol-induced seizures in rats with an ED50 of 6.2 mg/kg (Loscher, W., Honack, D., Fassbender, C.P., & Nolting, B. (1991). The role of technical, biological and pharmacological factors in the laboratory evaluation of anticonvulsant drugs. III. Pentylenetetrazole seizure models. *Epilepsy Res.* **8**: 171-189), retigabine was also active in the carageenan model of inflammatory pain with an ED50 of 4.5 mg/kg (Clayton, N.M., Oaklety, I., Thompson, S., Wheeldon, A. Sargent, B. & Bountra, C. (1997). Validation of the dual weight averager as an instrument for the measurement of clinically relevant pain. *Br. J. Pharmacol*. 120: P78). Based on this result it is extremely likely that an opener of KCNQ2/3 will be active in pain.

Retigabine and pharmaceutically acceptable salts and solvates thereof may be prepared, formulated and administered according to the methods described in USP5,384,330, incorportaed herein by reference.

KCNQ2/3 potassium channel openers, including retigabine, may, for example, be formulated for oral, buccal, parenteral, depot or rectal administration or in a form suitable for administration by inhalation or insufflation (either through the mouth or nose). Oral and parenteral formulations are preferred.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or lozenges formulated in conventional manner.

KCNQ2/3 potassium channel openers may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

KCNQ2/3 potassium channel openers may also be formulated in rectal compositions such as suppositories or retention enemas, e.g. containing conventional suppository bases such as cocoa butter or other glycerides.

KCNQ2/3 potassium channel openers may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, KCNQ2/3 potassium channel openers may be formulated as solutions for administration via a suitable metered or unit dose device or alternatively as a powder mix with a suitable carrier for administration using a suitable delivery device.

Suitable dose ranges are described in the art, that is to say that for use in conditions according to the present invention the compounds may be used at doses appropriate for other conditions for which KCNQ2/3 compounds, including retigabine, are known to be useful. It will be appreciated that it may be necessary to make routine variations to the dosage, depending on the age and condition of the patient, and the precise dosage will be ultimately at the discretion of the attendant physician or veterinarian. The dosage will also depend on the route of administration and the particular compound selected. A suitable dose range is for example 0.01 to 1000 mg/kg, such as from 0.1 to about 200 mg/kg, e.g. 0.1 mg/kg to 10 mg/kg, bodyweight per day.

The KCNQ2/3 potassium channel openers useful in the instant invention may, if desired, be administered in combination with one or more other therapeutic agents and formulated for administration by any convenient route in a conventional manner. Appropriate doses will be readily appreciated by those skilled in the art.

## Claims

1. The use of retigabine in the preparation of a medicament for: use in functional bowel disorders; neurodegenerative diseases; neuroprotection; tinnitus; preventing or reducing dependence on, or preventing or reducing tolerance or reverse tolerance to, a dependence - inducing agent; cancerous diseases; inflammatory processes; ophthalmic diseases; cognitive disorders; and migraine; and use as a centrally acting analgesic.

2. Use as claimed in claim 1 for the preparation of a medicament for use as a centrally acting analgesic.

3. The use of retigabine in the preparation of a medicament for use in migraine or the pain associated with migraine
